# EUROPEAN PATENT SPECIFICATION

(11) **EP 1 586 320 B1**
(45) Date of publication and mention of the grant of the patent: **16.07.2008**
(21) Application number: 05472001.6
(22) Date of filing: 02.02.2005
(51) Int. Cl.: A61K 31/435

(54) **Cytisine containing solid dosage form**
Cytisinehaltiges Arzneimittel
Un médicament contenant de la cytisine

(30) Priority: 16.04.2004 BG 10868604
(43) Date of publication of application: 19.10.2005
(73) Proprietor: Sopharma AD, 1220 Sofia (BG)
(72) Inventor: Daskalov, Vesselin Evgeniev, Sofia (BG); Timchev, Angel Radoslavov, Sofia 1231 (BG); Pavlova, Violeta Andreeva, Sofia (BG); Grozdeva, Radka Kostova, Sofia (BG); Tonev, Vlayko Petrov, Sofia 1574 (BG); Borissova, Yanka Ivanova, Sofia 1379 (BG); Dobreva, Dobrina Hristova, Sofia (BG); Bendurska, Todorka Boncheva, Sofia 1836 (BG)

(56) References cited:
- EP-A- 0 870 768
- EP-A- 1 178 045

## Description

### FIELD OF THE INVENTION

The invention relates to a cytisine containing solid dosage form for oral administration.

### BACKGROUND OF THE INVENTION

There is a known anti-smoking drug preparation in the form of coated tablets indicating trade name of Tabex (Pharmaceutical Catalogue of Drugs Allowed for Use in Bulgaria, 1998), which contains 1.5 mg per tablet of cytisine, and the following excipients: calcium dihydrogen phosphate, lactose, wheat starch, Avicel PH 101, talc powder, magnesium stearate, and coating. The tablet kernel composition does not provide stability of the coated tablets; neither does it provide a sufficient mechanical strength and the necessary even distribution of the active substance. The resulting tablets present low mechanical strength and low abrasion-resistance and do not meet the requirements set out in the European Pharmacopoeia regarding even distribution and solubility of cytisine.

A major problem resulting from the low concentration of the active substance is how to achieve an even distribution thereof throughout the tablet mass. The solution to this problem should involve obtaining a stable solid dosage form and a high level of solubility of the drug substance.

### TECHNICAL DESCRIPTION OF THE INVENTION

According to this invention, a solid dosage form has been obtained, the said form including the following contents: cytisine, lactose, microcrystal cellullose, talc powder, magnesium stearate and a coating, the lactose / microcrystal cellulose ratio being from 1 : 2.0 to 1: 2.5 and their total content in the cytisine tablets ranging from 87.0 to 97.0 percent of the tablet mass. The cytisine content of the tablets varies between 0.5 to 9.0 percent, the average dimension of the particles being 90 µm. The lactose is preferably a monohydrate with particle dimensions up to 500 µm. The average dimension of the microcrystal cellulose particles is 90 µm. The compositions of the present invention contain, as a lubricant, magnesium stearate in amounts ranging from 0.5 to 1.0 percent of the total tablet mass and talc powder as a glidant agent, its amounts ranging from 1.0 to 3.0 percent of the total tablet mass.

With this composition of the solid dosage form is achieved an even distribution of the active substance throughout the tablet mass. In testing the non-coated tablets, no deviations of the cytisine content have been found beyond ± 15.0-percent of the average cytisine content. The resulting tablets possess a level of mechanical strength ranging between 60 and 90 N, a solubility level not lower than 70 percent after 45 minutes of time and good disintegration. The final tablets meet the state-of-the art pharmacopoeic standards. The exclusion of two excipients - wheat starch and calcium hydrogen phosphate - lowers production costs.

This composition of the solid dosage form is illustrated, not exclusively, by the following examples where the amounts of the substances per tablet are given in mg:

| | Example 1 | Example 2 |
|---|---|---|
| Cytisine | 1.5 | 1.5 |
| Lactose | 31.833 | 27.290 |
| Microcrystal cellulose | 63.667 | 68.210 |
| Talc powder | 2.0 | 2.0 |
| Magnesium stearate | 1.0 | 1.0 |
| Film coating | 3.0 | 3.0 |
| Lactose/ microcrystal cellulose ratio | 1:2 | 1:2.5 |

The achievement of a high level of cytisine homogeneity in the tablet mass, in combination to high stability and the required solubility of the active substance are illustrated by the following examples of testing uncoated tablets and the final film-coated tablets. Two lots of tablets made according example 2 have been tested as follows:

### 1. Testing of uncoated tablets:

- Disintegration of an uncoated tablet
Norm: not longer than 15 min

| | |
|---|---|
| Lot No | X |
| 051099 | 1 |
| 020200 | 1 |

- Mechanical strength
Norm : 60-90 N

| No | Lot No 051099 | Lot No 020200 |
|---|---|---|
| 1 | 85 | 73 |
| 2 | 83 | 82 |
| 3 | 89 | 71 |
| 4 | 89 | 77 |
| 5 | 78 | 74 |
| 6 | 95 | 84 |
| 7 | 93 | 77 |
| 8 | 94 | 72 |
| 9 | 85 | 86 |
| 10 | 85 | 85 |
| 11 | 81 | 79 |
| 12 | 89 | 79 |
| 13 | 78 | 82 |
| 14 | 86 | 67 |
| 15 | 97 | 81 |
| 16 | 88 | 76 |
| 17 | 90 | 81 |
| 18 | 81 | 77 |
| 19 | 85 | 83 |
| 20 | 88 | 80 |

- Cytisine content per uncoated tablet
Norm: 1.387-1.612 mg

| Lot No | Cytisine mg |
|---|---|
| 051099 | 1.55 |
| 020200 | 1.49 |

### 2. Testing film- coated tablets

| STABILITY TEST TABLE | | | | | | | | | |
|---|---|---|---|---|---|---|---|---|---|
| applied to film coated tablets containing 1.5 mg of cytisine | | | | | | | | | |
| Lost tested: 051099 and 020200 | | | | | | | | | |
| Storage conditions: Temperature (25 ± 2)°C; Relative humidity (60 ± 5)% | | | | | | | | | |
| Package: Original PVC/Aluminium foil, dry and dark place, at a temperature under 25°C | | | | | | | | | |
| Analytical method: In compliance with the Company Standard N 007040154-02 | | | | | | | | | |
| Storage time and conditions | Appearance | Colour | Disintegration time (min) | Solubility of cytisine (percentage) after 45 min | Total content of similar substances (percentage) as related to cytisine | N-metyl-cytisinee (percentage) as related to cytisinee | N-formyl-cytisinee (percentage) as related to cytisinee | Cytisine content of one coalted tablet | Microbial content |
| 1 | 2 | 3 | 4 | 5 | 6 | 7 | 8 | 9 | 10 |
| Lot 051099 | | | | | | | | | |
| Initial analysis | Coated tablets having the form of a perfect disk 6 mm in diameter | Beige | 6 | 98 | 0.28 | 0.13 | 0.12 | 1.57 | fit |
| 9 months | fit | fit | 6 | 96 | 0.30 | 0.14 | 0.14 | 1.55 | fit |
| 12 months | fit | fit | 7 | 95 | 0.30 | 0.14 | 0.14 | 1.55 | fit |
| 18 months | fit | fit | 7 | 94 | 0.32 | 0.14 | 0.14 | 1.55 | fit |
| 24 months | fit | fit | 8 | 93 | 0.32 | 0.15 | 0.15 | 1.52 | fit |
| 30 months | fit | fit | 10 | 90 | 0.34 | 0.15 | 0.16 | 1.52 | fit |

| Lot 020200 | | | | | | | | | |
|---|---|---|---|---|---|---|---|---|---|
| Initial analysis | Coated tablets having the form of a perfect disk 6mm in diameter | Beige | 7 | 99 | 0.24 | 0.12 | 0.10 | 1.50 | fit |
| 9 months | fit | fit | 7 | 97 | 0.25 | 0.14 | 0.10 | 1.49 | fit |
| 12 months | fit | fit | 8 | 96 | 0.28 | 0.16 | 0.10 | 1.47 | fit |
| 18 months | fit | fit | 8 | 95 | 0.28 | 0.18 | 0.11 | 1.47 | fit |
| 24 months | fit | fit | 9 | 92 | 0.33 | 0.18 | 0.12 | 1.45 | fit |
| 30 months | fit | fit | 10 | 89 | 0.38 | 0.18 | 0.12 | 1.45 | fit |

## Claims

1. Cytisine solid dosage form including lactose, microcrystal cellulose, talc powder, magnesium stearate, and coating, **characterised by the fact of** containing from 0.5 to 9.0 percent of cytisine, as well lactose and microcrystal cellulose in a ratio of 1:2.0 up to 1:2.5, their total content ranging from 87.0 to 97.0 percent of the tablet mass.

2. Solid dosage form according to Claim 1, **characterised by** the fact that the average dimension of the cytisine particles is 90 µm.

3. Solid dosage form according to Claim 1, **characterised by** the fact that the lactose is a monohydrate lactose with particle dimensions up to 500 µm.

4. Solid dosage form according to Claim 1, characterise by the fact that the microcrystal cellulose particles have an average dimension of 90 µm.

## Patentansprüche

1. Arzneiform des Cytisine, einschliessend Lactose, mikrokristalle Cellulose, Talk, Magnesiumstearat und Filmübezug, **dadurch gekennzeichnet, dass** diese von 0,5 bis 9,0% Cytisine, Lactose und mikrokristalle Cellulose im Verhältniss von 1: 2 bis 1: 2,5 enthält, wobei deren Gesamtgehalt von 87,0 bis 97,0 % bezüglich der Masse der Tablette ist.

2. Arzneiform nach Anspruch 1, **dadurch gekennzeichnet, dass** das Cytisine mit durchschnittlichen Mass der Teilchen 90 µ*m* ist

3. Arzneiform nach Anspruch 1, **dadurch gekennzeichnet, dass** die Lactose Lactosemonohydrat ist mit Mass der Teilchen bis 500 µ*m.*

4. Arzneiform nach Anspruch 1, **dadurch gekennzeichnet, dass** die mikrokristalle Cellulose mit durchschnittlichen Mass der Teilchen 90 µ*m* ist

## Revendications

1. Composition pharmaceutique solide de Cytisine comprenant de la lactose, de la cellulose microcristalline, du talc, du stéarate de magnésium et un enrobage, **caractérisée en ce qu'**elle contient entre 0,5 et 9,0 % de Cytisine, de la lactose et de la cellulose microcristalline en proportion de 1:2,0 à 1:2,5 alors que leur contenu total est entre 87,0 et 97,0 % de la masse de la tablette.

2. Composition pharmaceutique solide selon la revendication 1, **caractérisée en ce que** la dimension moyenne des particules de Cytisine est 90 µm.

3. Composition pharmaceutique solide selon la revendication 1, **caractérisée en ce que** la lactose est une lactose monohydratée avec une dimension des particules jusqu'à 500 µm.

4. Composition pharmaceutique solide selon la revendication 1, **caractérisée en ce que** la dimension moyenne des particules de la cellulose microcristalline est égale à 90 µm.
